# EUROPEAN PATENT APPLICATION

(11) **EP 4 559 580 A1**
(43) Date of publication of application: **28.05.2025**
(21) Application number: 23842851.0
(22) Date of filing: 07.07.2023
(51) Int. Cl.: B03C 5/00, C12M 1/00, G01N 37/00

(54) **FLOW PATH CHIP**

(30) Priority: 21.07.2022 JP 2022116506
(71) Applicant: SCREEN Holdings Co., Ltd., Kyoto-shi, Kyoto 602-8585 (JP)
(72) Inventor: URAKAWA Satoshi, Kyoto-shi, Kyoto 602-8585 (JP); SANADA Masakazu, Kyoto-shi, Kyoto 602-8585 (JP)
(74) Representative: Goddar, Heinz J.
(86) International application number: PCT/JP2023/025289
(87) International publication number: WO 2024/018926

(57) **Abstract**

A channel chip (1) includes a plurality of filter layers (40), a first common supply passage (5), a common channel (7), and a dielectrophoretic layer (50). The plurality of filter layers (40) each have an HDF (3). The first common supply passage (5) supplies a sample liquid (L1) to the HDFs (3) of the plurality of filter layers (40). The liquid that has passed through the plurality of HDFs (3) passes through the common channel (7). The dielectrophoretic layer (50) includes an electrode (51) and an electrode (53) for causing dielectrophoresis of dielectric particles contained in a liquid passing through the common channel (7). The plurality of filter layers (40) and the dielectrophoretic layer (50) are stacked.

## Description

### Technical Field

The present invention relates to a channel chip.

### Background Art

Conventionally, a separation device that separates specific cells from blood has been known (See, for example, Patent Literature 1.). Patent Literature 1 discloses a channel chip including a replacement portion that extracts cells having a predetermined size or larger from blood, and a separation portion that separates cancer cells from a plurality of types of cells that has passed through the replacement portion by means of a dielectrophoretic force.

### Citation List

### Patent Literature

Patent Literature 1: Japanese Patent Application Publication No. 2017-134020

### Summary of Invention

### Technical Problem

In general, in a channel chip like that described in Patent Literature 1, the processing capacity is as low as several tens to several hundreds µL/min. For this reason, a channel chip capable of processing a larger amount of sample liquid is desired.

The present invention has been made in view of the above problems, and an object of the present invention is to provide a channel chip capable of processing more sample liquid.

### Solution to Problem

A channel chip according to one aspect of the present invention includes a plurality of filter layers, a common supply passage, a common channel, and a dielectrophoretic layer. Each of the plurality of filter layers has a hydrodynamic filter. The common supply passage supplies a sample liquid to the hydrodynamic filters of the plurality of filter layers. A liquid that has passed through the plurality of hydrodynamic filters passes through the common channel. The dielectrophoretic layer has electrodes that cause dielectrophoresis of dielectric particles contained in the liquid passing through the common channel. The plurality of filter layers and the dielectrophoretic layer are stacked.

In a mode of the present invention, the plurality of filter layers may include a first filter layer stacked on the dielectrophoretic layer and one or more second filter layers stacked on the first filter layer. The common channel may be disposed in the first filter layer.

In a mode of the present invention, the plurality of filter layers may include a plurality of the second filter layers. The channel chip may include a plurality of outlet-side connection channels connecting outlets of the hydrodynamic filters of the plurality of second filter layers and the common channel. The plurality of outlet-side connection channels may extend to the first filter layer and be connected to the common channel without joining together in the second filter layers.

In a mode of the present invention, each of the filter layers may have the plurality of hydrodynamic filters.

In a mode of the present invention, the plurality of hydrodynamic filters of each of the filter layers may be disposed symmetrically with respect to the common channel.

In a mode of the present invention, the channel chip may include a plurality of inlet-side connection channels that connect the common supply passage and inlets of the plurality of hydrodynamic filters in each of the filter layers. In each of the filter layers, the plurality of inlet-side connection channels may have substantially the same channel length.

In a mode of the present invention, the channel chip may include a first recovery passage and a pair of second recovery passages connected to a downstream portion of the common channel. The pair of second recovery passages may be disposed such as to sandwich the first recovery passage. The dielectric particles separated from the liquid passing through the hydrodynamic filter may flow through the first recovery passage.

### Advantageous Effects of Invention

According to the present invention, it is possible to provide a channel chip capable of processing more sample liquid.

### Brief Description of Drawings

[FIG. 1] is a cross-sectional view schematically illustrating a structure of a separation device including a channel chip according to a preferred embodiment of the present invention.
[FIG. 2] is a perspective view schematically illustrating a structure in which a channel chip is disassembled into a plurality of filter layers and a dielectrophoretic layers.
[FIG. 3] is a plan view schematically illustrating a structure of a first filter layer.
[FIG. 4] is a plan view schematically illustrating a structure of a second filter layer.
[FIG. 5] is a plan view schematically illustrating a structure of a hydrodynamic filter.
[FIG. 6] is a plan view schematically illustrating a structure around a common channel.
[FIG. 7] is a plan view schematically illustrating a structure of the dielectrophoretic layer.
[FIG. 8] is a plan view schematically illustrating a structure of a common channel and electrodes.
[FIG. 9] is a plan view schematically illustrating a structure of the channel chip.
[FIG. 10] is a plan view schematically illustrating a structure around a branch portion of the common channel.
[FIG. 11] is a block diagram illustrating the configuration of a separation device.
[FIG. 12] is a cross-sectional view schematically illustrating a structure of a separation device including a channel chip according to a first modified example of the present invention.
[FIG. 13] is a plan view schematically illustrating a structure of a first filter layer of a channel chip according to a second modified example of the present invention.

### Description of Embodiments

Hereinafter, a preferred embodiment of the present invention will be described with reference to the drawings. It is noted that in the drawings, the same or corresponding portions will be given the same reference signs and will not be repeatedly described.

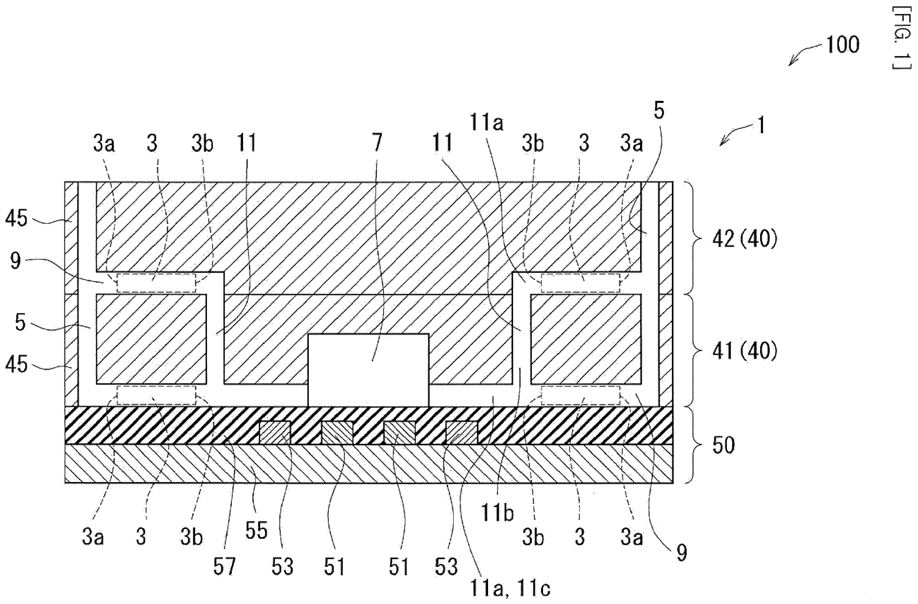

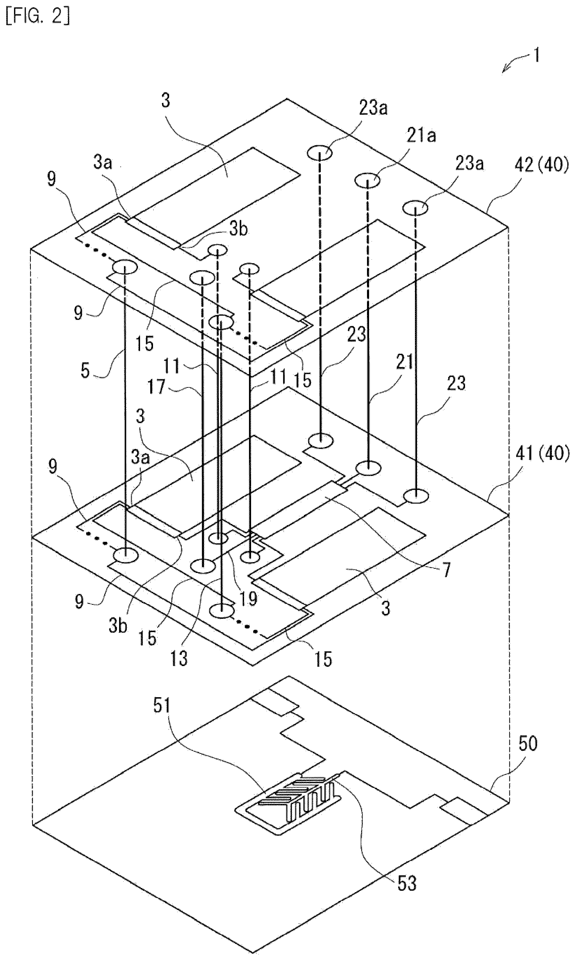

A separation device 100 including a channel chip 1 according to a preferred embodiment of the present disclosure will be described with reference to FIGS. 1 to 11. Fig. 1 is a cross-sectional view schematically illustrating a structure of the separation device 100 including the channel chip 1 according to a preferred embodiment of the present invention. FIG. 2 is a perspective view schematically illustrating a structure in which the channel chip 1 is disassembled into a plurality of filter layers 40 and a dielectrophoretic layer 50.

As illustrated in FIGS. 1 and 2, the separation device 100 of the present preferred embodiment includes the channel chip 1. The channel chip 1 includes the plurality of filter layers 40 and the dielectrophoretic layer 50. Each of the plurality of filter layers 40 has hydrodynamic filters 3 (Hydrodynamic filtration: HDF) (hereinafter referred to as the HDFs 3). In order to simplify the drawing, the HDF 3 is indicated by rectangular broken lines in FIG. 1. For example, the channel chip 1 is a microchannel chip for the purpose of separating and concentrating fine particles. For example, the channel chip 1 can separate specific cells from blood as described later.

In the present preferred embodiment, the plurality of filter layers 40 and the dielectrophoretic layer 50 are stacked. Therefore, since the plurality of HDFs 3 are disposed for one channel chip 1, it is possible to process more sample liquid L1 described later. Also, it is not necessary to provide the dielectrophoretic layer 50 for each of the plurality of HDFs 3. That is, it is not necessary to provide the same number of dielectrophoretic layers 50 as the number of HDFs 3. Therefore, it is possible to suppress an increase in the size of the channel chip 1 or an increase in the size of the separation device 100.

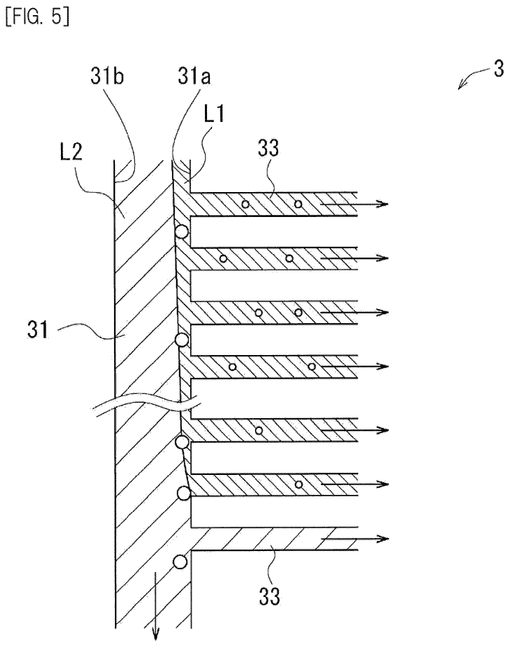

The HDF 3 separates particles larger than a predetermined particle size from the passing sample liquid L1 (see FIG. 5). It is noted that the detailed structure of the HDF 3 will be described later.

The dielectrophoretic layer 50 includes an electrode 51 and an electrode 53. The electrode 51 and the electrode 53 are electrodes for dielectrophoresis (DEP) of dielectric particles contained in a liquid passing through a common channel 7 described later. It is noted that the detailed structure of the dielectrophoretic layer 50 will be described later.

Also, the plurality of filter layers 40 include a first filter layer 41 stacked on the dielectrophoretic layer 50 and one or more second filter layers 42 stacked on the first filter layer 41. It is noted that, in the present preferred embodiment, the common channel 7 to be described later is disposed in the first filter layer 41. Therefore, since the dielectrophoretic layer 50 can be disposed near the common channel 7, the dielectrophoretic force can be easily applied to the dielectric particles contained in the liquid passing through the common channel 7. Also, the second filter layer 42 can be easily stacked as compared with a case where the first filter layer 41 is disposed on one surface of the dielectrophoretic layer 50 and the second filter layer 42 is disposed on the other surface of the dielectrophoretic layer 50. In other words, the channel chip 1 can be easily manufactured as compared with a case where the plurality of filter layers 40 are disposed such as to sandwich the dielectrophoretic layer 50.

The channel chip 1 further includes a first common supply passage 5 and the common channel 7. It is noted that the first common supply passage 5 is an example of the "common supply passage" of the present invention. The first common supply passage 5 extends in a stacking direction of the filter layer 40 and the dielectrophoretic layer 50. The first common supply passage 5 supplies the sample liquid L1 to the HDF 3 of the plurality of filter layers 40. It is noted that, although two first common supply passages 5 are illustrated in FIG. 1 in order to facilitate understanding, only one first common supply passage 5 may be provided as illustrated in FIG. 2. Hereinafter, an example in which only one first common supply passage 5 is provided will be described. Also, an outlet 3b of the HDF 3 of the plurality of filter layers 40 is connected to the common channel **7.** The liquid that has passed through the plurality of HDFs 3 flows through the common channel 7.

In the present preferred embodiment, each of the filter layers 40 has a plurality of the HDFs 3 (here, two HDFs 3). Therefore, as compared with a case where each of the filter layers 40 has only one HDF 3, one channel chip 1 can process more sample liquid L1.

The plurality of HDFs 3 of each filter layer 40 are disposed symmetrically with respect to the common channel 7. Therefore, the design of each filter layer 40 can be simplified. Also, among the plurality of HDFs 3 of each filter layer 40, the dimensions (the lengths, widths, and depths) of channels (main channels 31 and auxiliary channels 33 to be described later) constituting the HDFs 3 are formed to be the same as each other. In the present preferred embodiment, in all filter layers 40, the channels constituting the HDF 3 are formed to have the same dimensions.

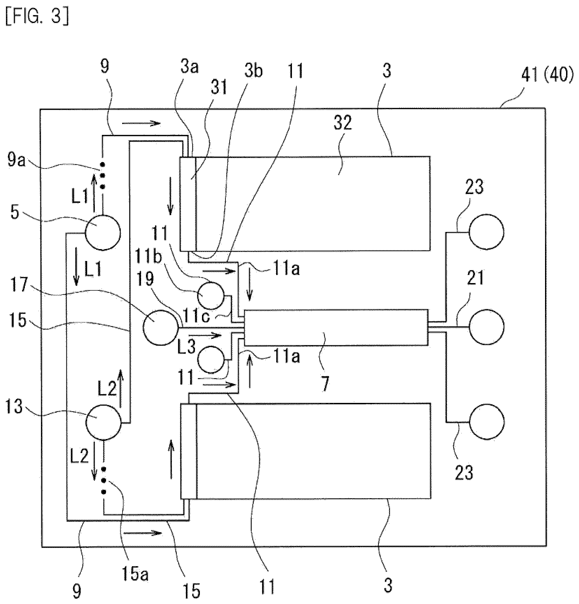

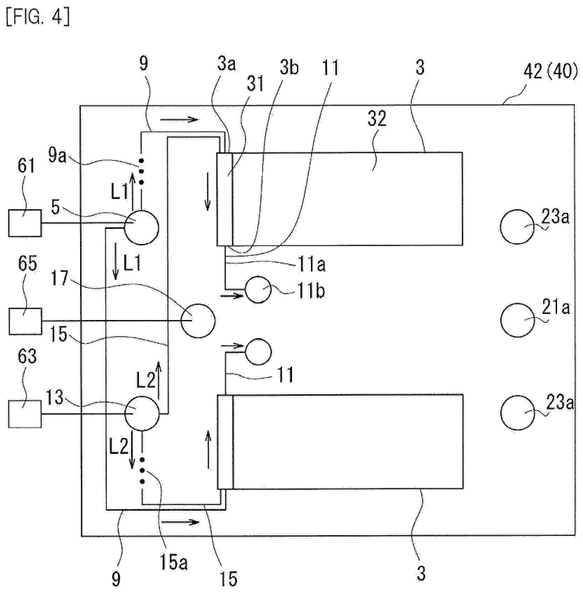

FIG. 3 is a plan view schematically illustrating a structure of the first filter layer 41. FIG. 4 is a plan view schematically illustrating a structure of the second filter layer 42. As illustrated in FIGS. 3 and 4, the channel chip 1 further includes a first connection channel 9 and a second connection channel 11. It is noted that the first connection channel 9 is an example of the "inlet-side connection channel" of the present invention. Also, the second connection channel 11 is an example of the "outlet-side connection channel" of the present invention.

The first connection channel 9 connects the first common supply passage 5 and an inlet 3a of the HDF 3. Specifically, the channel chip 1 includes a plurality of first connection channels 9 in each filter layer 40. In each filter layer 40, channel lengths from the first common supply passage 5 to the inlets 3a of the plurality of HDFs 3 are substantially the same. That is, in each filter layer 40, the channel lengths of a plurality of the first connection channels 9 (here, two first connection channels 9) are substantially the same. Therefore, when the sample liquid L1 is supplied to the first common supply passage 5, the sample liquid L1 flows through the plurality of HDFs 3 substantially simultaneously in each filter layer 40. Therefore, since the sample liquid L1 flows under substantially the same condition with respect to the plurality of HDFs 3, it is possible to suppress variation in the separation performance by the HDFs 3.

It is noted that, although at least one first connection channel 9 has a bypass portion 9a in order to make the channel lengths of the plurality of first connection channels 9 substantially the same, the bypass portion 9a is drawn with three dots in order to simplify the drawing. Also, in each filter layer 40, the cross-sectional dimensions such as the widths and depths of the plurality of first connection channels 9 are substantially the same. It is noted that, among the plurality of filter layers 40, the channel lengths and the cross-sectional dimensions of the first connection channels 9 may be substantially the same, or may be different in consideration of channel resistance or the like.

The second connection channel 11 connects the outlet 3b of the HDF 3 and the common channel 7. Specifically, the second connection channel 11 connecting the outlet 3b of the HDF 3 of the first filter layer 41 and the common channel 7 is constituted by a first portion 11a extending in a planar direction (the direction orthogonal to the stacking direction) from the outlet 3b of the HDF 3. The second connection channel 11 connecting the outlet 3b of the HDF 3 of the second filter layer 42 and the common channel 7 includes the first portion 11a extending in the plane direction from the outlet 3b of the HDF 3, a second portion 11b extending in the stacking direction from the first portion 11a, and a third portion 11c extending in the plane direction from the second portion 11b and connected to the common channel 7. It is noted that the first portion 11a and the third portion 11c are illustrated as a common portion on the first filter layer 41 in FIG. 1 in order to simplify the drawing.

The channel chip 1 further includes a second common supply passage 13 and a third connection channel 15. It is noted that the second common supply passage 13 and the third connection channel 15 are omitted in FIG. 1 in order to simplify the drawing. As illustrated in FIGS. 2 to 4, the second common supply passage 13 extends in the stacking direction of the filter layer 40 and the dielectrophoretic layer 50. The second common supply passage 13 supplies a transport liquid L2 (see FIG. 5) to the HDF 3 of the plurality of filter layers 40. The third connection channel 15 connects the second common supply passage 13 and the inlet 3a of the HDF 3.

In each filter layer 40, the channel lengths from the second common supply passage 13 to the inlets 3a of the plurality of HDFs 3 are substantially the same. That is, in each filter layer 40, the channel lengths of a plurality of the third connection channels 15 (here, two third connection channels 15) are substantially the same. It is noted that, although at least one third connection channel 15 has a bypass portion 15a in order to make the channel lengths of the plurality of third connection channels 15 substantially the same, the bypass portion 15a is drawn with three dots in order to simplify the drawing. Also, in each filter layer 40, the cross-sectional dimensions such as the widths and depths of the plurality of third connection channels 15 are substantially the same. It is noted that, among the plurality of filter layers 40, the channel lengths and the cross-sectional dimensions of the third connection channels 15 may be substantially the same, or may be different in consideration of channel resistance or the like.

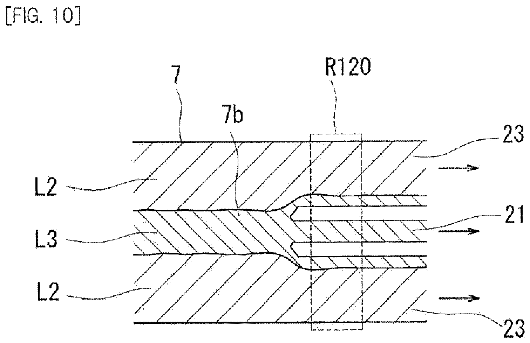

The channel chip 1 further includes a recovered liquid supply passage 17 and a fourth connection channel 19. It is noted that the recovered liquid supply passage 17 and the fourth connection channel 19 are omitted in FIG. 1 in order to simplify the drawing. The recovered liquid supply passage 17 supplies a recovered liquid L3 (see FIG. 10) to the common channel 7. The recovered liquid L3 is not particularly limited but is, for example, the same in components as the transport liquid L2. It is noted that the recovered liquid L3 may be different in components than the transport liquid L2. The fourth connection channel 19 is disposed in the first filter layer 41. The fourth connection channel 19 connects the recovered liquid supply passage 17 and the common channel 7.

Next, the cross-sectional structure of the channel chip 1 will be described with reference to FIG. 1. As illustrated in Fig. 1, the dielectrophoretic layer 50 further includes a substrate 55 and an insulating protective film 57 in addition to the electrode 51 and the electrode 53. The substrate 55 is not particularly limited but is made of, for example, glass. In the present preferred embodiment, the substrate 55 is made of quartz glass. Also, the substrate 55 is a substantially rectangular flat plate, for example.

The electrode 51 and the electrode 53 are disposed on one surface of the substrate 55. The electrode 51 and the electrode 53 are made of metal such as aluminum or copper, for example. The electrode 51 and the electrode 53 are formed in a predetermined shape by, for example, a vacuum deposition method, a photolithography method, and an etching method.

The insulating protective film 57 covers the electrode 51, the electrode 53, and the substrate 55. The insulating protective film 57 is not particularly limited but is, for example, an oxide film such as a silicon oxide film or a silicon nitride film. The insulating protective film 57 suppresses, for example, time degradation of the electrode 51 and the electrode 53 due to moisture.

The filter layer 40 further includes insulation layers 45. The insulation layer 45 is made of, for example, resin. In the present preferred embodiment, the insulation layer 45 is transparent. The insulation layer 45 is formed from, for example, dimethylpolysiloxane (PDMS). In the insulation layer 45, the HDF 3 and various channels are formed. The plurality of filter layers 40 are formed, for example, by sequentially stacking, on the dielectrophoretic layer 50, the plurality of insulation layers 45 in which the HDFs 3 and various channels are formed. It is noted that the filter layer 40 may be formed, for example, by stacking an uncured resin on the dielectrophoretic layer 50 and curing the resin.

Next, the structure of the HDF 3 will be described in detail with reference to FIG. 5. FIG. 5 is a plan view schematically illustrating a structure of the HDF 3. As illustrated in FIG. 5, the HDF 3 includes the main channel 31 and a plurality of the auxiliary channels 33.

The main channel 31 extends in a straight-line form toward, for example, a predetermined direction. A sample liquid L1 flows through the main channel 31. In the present preferred embodiment, the sample liquid L1 and the transport liquid L2 flow through the main channel 31. The sample liquid L1 is a liquid containing fine particles. The sample liquid L1 is not particularly limited but is blood, for example. The transport liquid L2 is a liquid not containing fine particles, for example. The transport liquid L2 is not particularly limited but is a liquid culture media, for example.

The plurality of auxiliary channels 33 branch from the main channel 31. Specifically, the plurality of auxiliary channels 33 are disposed at substantially equal pitches along the direction in which the main channel 31 extends, for example. The plurality of auxiliary channels 33 have the same length, for example. The plurality of auxiliary channels 33 extend in a direction intersecting the main channel 31. In the present preferred embodiment, the plurality of auxiliary channels 33 extend in a direction orthogonal to the main channel 31.

Also, the plurality of auxiliary channels 33 are formed so that channel resistance is gradually reduced from the upstream side of the main channel 31 toward the downstream side. Specifically, in the present preferred embodiment, a portion on the proximal end side (a side close to the main channel 31) of each of the auxiliary channels 33 is formed to have a first width. Meanwhile, a portion of each auxiliary channel 33 which is located on the terminal side (the side far from the main channel 31) is formed to have a second width larger than the first width. Then, the position where the width of the auxiliary channel 33 changes from the first width to the second width is disposed closer to the proximal end side as the auxiliary channel is closer to the downstream side. The first width is not particularly limited but is, for example, about 10 µm to several 10 µm. The second width is not particularly limited but is about several 10 µm to 100 µm, for example. It is noted that all of the auxiliary channels 33 may be formed to have the same constant width. In this case, for example, the auxiliary channel 33 closer to the downstream side may be formed shorter.

Part of the liquid flowing through the main channel 31 flows into the plurality of auxiliary channels 33. Also, part of the particles contained in the liquid flowing through the main channel 31 flows into the plurality of auxiliary channels 33. Whether or not the particles flow into the plurality of auxiliary channels 33 depends on, for example, the particle size. The particles having the smaller particle sizes more easily flow into the auxiliary channel 33, and the particles having the larger particle sizes less easily flow into the auxiliary channel 33. For example, in a case where blood is used as the sample liquid L1, it is possible to make blood plasma (also called as plasma) serving as a liquid component of blood as well as red blood cells and blood platelets flow to the auxiliary channel 33 and inhibit cancer cells and white blood cells contained in blood from flowing to the auxiliary channel 33. That is, it is possible to separate the particles having the large particle sizes, such as cancer cells and the white blood cells, from blood.

Successively, with reference to FIGS. 3 to 5, an operating principle of the HDF 3 will be described. As illustrated in FIGS. 3 and 4, when the sample liquid L1 is supplied to the first common supply passage 5 and the transport liquid L2 is supplied to the second common supply passage 13, the sample liquid L1 and the transport liquid L2 flow through the main channel 31 via the first connection channel 9 and the third connection channel 15, respectively. As illustrated in FIG. 5, the sample liquid L1 flows along an inner wall 31a on the auxiliary channels 33 side, and the transport liquid L2 flows along an inner wall 31b on the opposite side to the auxiliary channels 33. At this time, centers of various particles pass through positions separated from the inner wall 31a of the main channel 31 by not less than radii of the particles.

Here, the flow speed changes according to the position of the main channel 31 in the width direction. Specifically, the flow speed decreases near the inner wall 31a and the inner wall 31b of the main channel 31. On the other hand, the flow speed is the largest at a position far from the inner wall 31a and the inner wall 31b of the main channel 31 (the center of the main channel 31 in the width direction).

For this reason, since the flow speed of particles having a small particle size and passing near the inner wall 31a is low, the particles easily flow into the auxiliary channels 33. On the other hand, since the flow speed of particles having a large particle size and passing through a position far from the inner wall 31a is high, the particles do not flow into the auxiliary channels 33 and travel straight through the main channel 31. That is, the particles larger than a predetermined size are not discharged from the main channel 31 to the auxiliary channel 33, whereas the particles of the predetermined size or smaller are discharged from the main channel 31 to the auxiliary channel 33. Since discharge from the main channel 31 to the auxiliary channels 33 is repeated by the plurality of auxiliary channels 33, the particles of the predetermined size or smaller do not reach the downstream end (the outlet 3b of the HDF 3) of the main channel 31. It is noted that blood plasma serving as a liquid component does not reach the downstream end of the main channel 31 as well. Therefore, it is possible to separate, for example, the particles having the large particle sizes, such as cancer cells and the white blood cells, from blood by the channel chip 1.

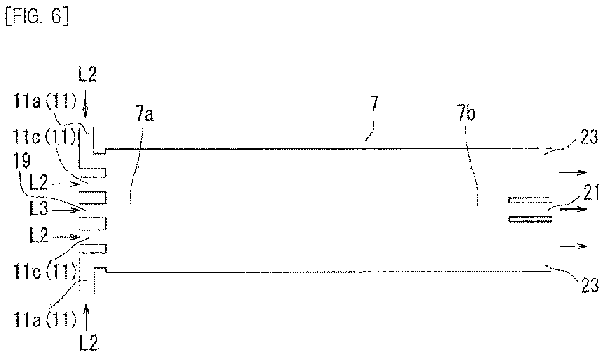

Next, the first filter layer 41 will be further described with reference to FIG. 6. FIG. 6 is a view schematically illustrating a structure around the common channel 7. As illustrated in FIG. 6, the common channel 7 is disposed in the first filter layer 41. The common channel 7 extends in a straight-line form toward a predetermined direction. The common channel 7 has a joining portion 7a and a branch portion 7b. The joining portion 7a is disposed at the upstream-side end portion of the common channel 7. The branch portion 7b is disposed at the downstream-side end portion of the common channel 7.

The second connection channel 11 is connected to the upstream end of the common channel 7. In the present preferred embodiment, four second connection channels 11 are connected to the upstream end of the common channel 7. Also, the fourth connection channel 19 is connected to the upstream end of the common channel 7. In the present preferred embodiment, the fourth connection channel 19 is disposed between the two second connection channels 11 and the two second connection channels 11. It is noted that the four second connection channels 11 are disposed symmetrically with respect to the fourth connection channel **19.** The second connection channel 11 and the fourth connection channel 19 join together at the joining portion 7a of the common channel 7.

The channel chip 1 further includes a first recovery passage 21 and a second recovery passage 23 which are connected to a downstream portion of the common channel 7. In the present preferred embodiment, the channel chip 1 includes the first recovery passage 21 connected to a downstream portion of the common channel 7 and a pair of the second recovery passages 23. The first recovery passage 21 and the pair of second recovery passage 23 are connected to the downstream end of the common channel 7. The common channel 7 branches into the first recovery passage 21 and the pair of second recovery passages 23 at the branch portion 7b. The pair of second recovery passages 23 are disposed such as to sandwich the first recovery passage 21. Therefore, as described later, when a target to be recovered (here, cancer cells) is recovered from the first recovery passage 21, the target to be recovered can be recovered from one first recovery passage 21. Therefore, the target to be recovered can be easily recovered as compared with the case where a pair of the first recovery passages 21 are provided.

As illustrated in FIG. 2, the first recovery passage 21 and the second recovery passage 23 are led out in the plane direction from the common channel 7 and then extend in the stacking direction. Also, the first recovery passage 21 and the second recovery passage 23 each have an outlet 21a and an outlet 23a. The outlet 21a and the outlet 23a are disposed in the second filter layer 42. The liquid that has passed through the first recovery passage 21 is recovered to the first recovery portion (not illustrated) via the outlet 21a. The first recovery portion includes a recovery container, for example. The liquid that has passed through the second recovery passage 23 is recovered to the second recovery portion (not illustrated) via the outlet 23a. The second recovery portion includes a recovery container, for example. It is noted that, in the present preferred embodiment, as will be described later, dielectric particles (here, cancer cells) separated from the liquid that has passed through the HDF 3 flow into the first recovery passage 21.

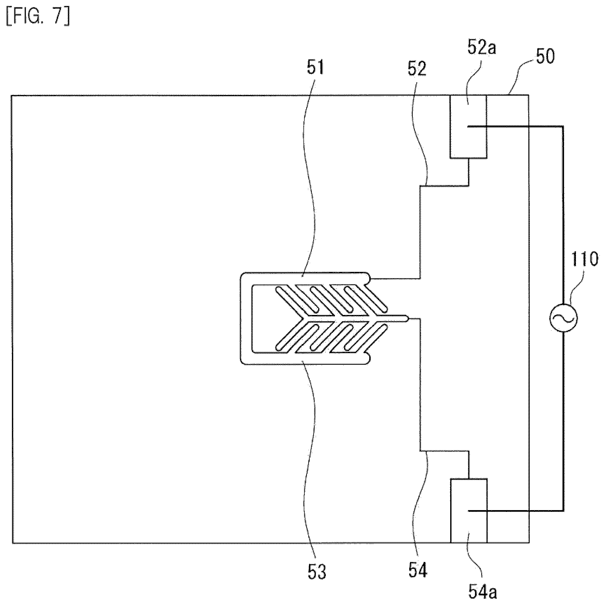

Next, separation by the dielectrophoretic layer 50 and dielectrophoresis will be described with reference to FIGS. 7 to 10. FIG. 7 is a plan view schematically illustrating a structure of the dielectrophoretic layer 50. As illustrated in FIG. 7, the dielectrophoretic layer 50 further includes a lead-out wiring 52 and a lead-out wiring 54 in addition to the electrode 51 and the electrode 53. One end of the lead-out wiring 52 is connected to the electrode 51. The lead-out wiring 52 has a terminal portion 52a. The terminal portion 52a is disposed at the other end of the lead-out wiring 52. The separation device 100 includes an AC source 110, and the terminal portion 52a of the lead-out wiring 52 is electrically connected to the AC source 110. One end of the lead-out wiring 54 is connected to the electrode 53. The lead-out wiring 54 has a terminal portion 54a. The terminal portion 54a is disposed at the other end of the lead-out wiring 54. The terminal portion 54a of the lead-out wiring 54 is electrically connected to the AC source 110. An AC voltage is applied between the terminal portion 52a and the terminal portion 54a by the AC source 110.

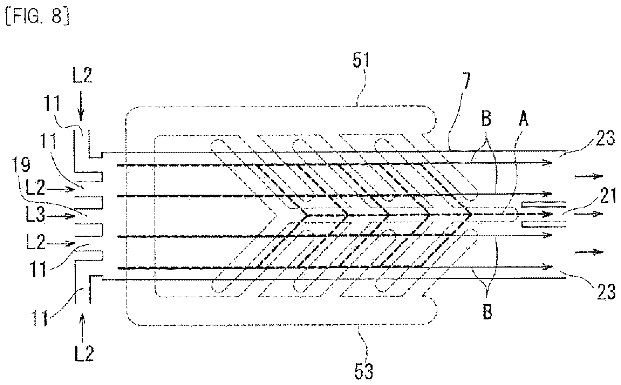

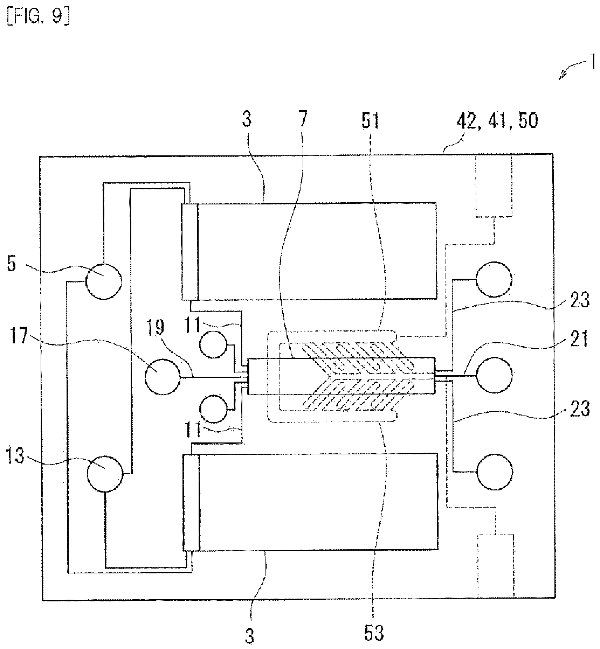

FIG. 8 is a plan view schematically illustrating a structures of the common channel 7, the electrode 51, and the electrode 53. FIG. 9 is a plan view schematically illustrating a structure of the channel chip 1. It is noted that, in order to facilitate understanding, the first filter layer 41 and the second filter layer 42 are indicated by solid lines and the dielectrophoretic layer 50 is indicated by broken lines in FIG. 9. The dielectrophoretic layer 50 can further separate particles flowing through the common channel 7 by dielectrophoresis. Specifically, as illustrated in FIGS. 8 and 9, the electrode 51 and the electrode 53 are disposed such as to overlap the common channel 7. That is, the electrode 51 and the electrode 53 face the common channel 7 in the stacking direction. The electrode 51 and the electrode 53 are teeth-shaped electrodes opposing each other. The teeth portions of the electrode 51 and the electrode 53 are inclined at, for example, 10° or more and 60° or less with respect to the common channel 7. In the present preferred embodiment, the electrode 51 and the teeth portion of the electrode 53 are disposed symmetrically in the width direction of the common channel 7. Specifically, the teeth portions of the electrode 51 and the electrode 53 extend from the outside in the width direction toward the center in the width direction of the common channel 7, from the upstream side toward the downstream side.

Applying an AC voltage between the electrode 51 and the electrode 53 by the AC source 110 can cause dielectrophoresis of the particles passing through the common channel **7.** Adjusting the frequency of the AC voltage to be applied makes it possible to separate and recover desired particles from a plurality of types of particles passing through the common channel 7 by dielectrophoresis.

Specifically, in a case where the plurality of types of particles passing through the common channel 7 contain dielectric particles, it is possible to separate particular dielectric particles from the plurality of types of particles by dielectrophoresis. For example, cancer cells and white blood cells are dielectric particles. In a case where the plurality of types of particles passing through the common channel 7 contain the cancer cells as well as particles other than the cancer cells, having a size larger than a predetermined size, (such as the white blood cells), AC voltage of a particular frequency is applied between the electrode 51 and the electrode 53 so that a dielectrophoretic force is made to easily act on the cancer cells, for example. As illustrated by FIG. 8, since this makes it possible to move the cancer cells along the electrode 51 and the electrode 53, it is possible to induce only the cancer cells to the first recovery passage 21 by dielectrophoresis. Therefore, it is possible to separate the cancer cells and other particles (such as the white blood cells). It is noted that the flow of cancer cells is indicated by an arrow A, and the flow of other particles is indicated by an arrow B in FIG. 8.

FIG. 10 is a plan view schematically illustrating a structure around the branch portion 7b of the common channel 7. More specifically, as illustrated in FIG. 10, the transport liquid L2 does not flow to the first recovery passage 21, and only the recovered liquid L3 flows. In the present preferred embodiment, the transport liquid L2 does not flow to the first recovery passage 21 but flows to the second recovery passage 23. The recovered liquid L3 flows to the first recovery passage 21 and the second recovery passage 23. Therefore, when no dielectrophoretic force is made to act, particles do not flow to the first recovery passage 21. On the other hand, in the present preferred embodiment, for example, cancer cells can be moved from the outside in the width direction to the inside in the width direction of the common channel 7 by making a dielectrophoretic force act. Therefore, it is possible to guide only cancer cells among the plurality of types of particles to the first recovery passage 21.

Next, the separation device 100 will be further described with reference to FIG. 4. As illustrated in FIG. 4, the separation device 100 further includes a sample liquid sending portion 61, a transport liquid sending portion 63, and a recovered liquid sending portion 65. The separation device 100 may include a control unit that drives the sample liquid sending portion 61, the transport liquid sending portion 63, and the recovered liquid sending portion 65. In the present preferred embodiment, a control unit 131 described later drives the sample liquid sending portion 61, the transport liquid sending portion 63, and the recovered liquid sending portion 65. The sample liquid sending portion 61, the transport liquid sending portion 63, and the recovered liquid sending portion 65 are not particularly limited but are, for example, micropumps. The micropumps may be mechanical pumps or may be non-mechanical pumps.

The sample liquid sending portion 61 is connected to the first common supply passage 5 and causes the sample liquid L1 to flow through the first common supply passage 5. When the sample liquid sending portion 61 supplies the sample liquid L1 to the first common supply passage 5, the sample liquid L1 flows to the first connection channel 9 and the HDF 3 of each filter layer 40.

The transport liquid sending portion 63 is connected to the second common supply passage 13 and causes the transport liquid L2 to flow to the second common supply passage 13. When the transport liquid sending portion 63 supplies the transport liquid L2 to the second common supply passage 13, the transport liquid L2 flows to the third connection channel 15 and the HDF 3 of each filter layer 40.

The recovered liquid sending portion 65 is connected to the recovered liquid supply passage 17 and causes the recovered liquid L3 to flow to the recovered liquid supply passage 17. When the recovered liquid sending portion 65 supplies the recovered liquid L3 to the recovered liquid supply passage 17, the recovered liquid L3 flows through the fourth connection channel 19 and the common channel 7.

In the present preferred embodiment, the sample liquid sending portion 61 supplies the sample liquid L1 so that the sample liquid L1 becomes a laminar flow. The transport liquid sending portion 63 supplies the transport liquid L2 so that the transport liquid L2 becomes a laminar flow. The recovered liquid sending portion 65 supplies the recovered liquid L3 so that the recovered liquid L3 becomes a laminar flow. Therefore, in the present preferred embodiment, each liquid becomes a laminar flow in the channel chip 1.

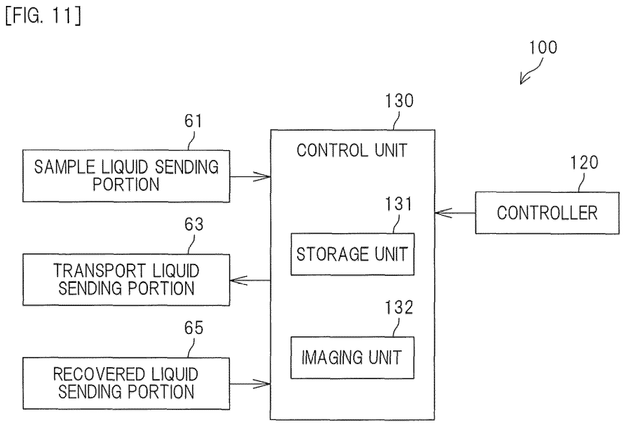

Next, the separation device 100 will be further described with reference to FIG. 11. FIG. 11 is a block diagram illustrating a configuration of the separation device 100. As illustrated in FIG. 11, the separation device 100 further includes an imaging unit 120 and a controller 130. The imaging unit 120 acquires an image of a predetermined region of the channel chip 1. The imaging unit 120 transmits captured image data to the control unit 131 (described later) of the controller 130. The imaging unit 120 includes, for example, a camera having an imaging element such as a CCD image sensor or a CMOS image sensor, and an optical microscope module. The imaging unit 120 may include other cameras or other microscopes. It is noted that the separation device 100 may further include a display unit that displays an image captured by the imaging unit 120.

The imaging unit 120 is used to check the operation of the channel chip 1. For example, in the present preferred embodiment, the imaging unit 120 is used to check whether or not the HDF 3 has separated particles larger than a predetermined particle size from particles having a predetermined particle size or smaller. Also, in the present preferred embodiment, the imaging unit 120 is used to check whether or not cancer cells and other particles are separated by dielectrophoresis.

Here, in the present preferred embodiment, as illustrated in FIG. 10, the first recovery passage 21 and the pair of second recovery passages 23 are disposed in an imaging region R120 of the imaging unit 120. Specifically, in the imaging region R120, the entire area of the first recovery passage 21 in the width direction and the entire area of the pair of second recovery passages 23 in the width direction are disposed. Therefore, the imaging unit 120 can image all particles passing through the first recovery passage 21 and all particles passing through the pair of second recovery passages 23. Therefore, the configuration of the separation device 100 can be simplified as compared with a case where one imaging unit 120 is provided for each recovery passage (the first recovery passage 21 and the pair of second recovery passages 23).

The controller 130 includes the control unit 131 and a storage unit 132. The control unit 131 includes a processor. The control unit 131 includes, for example, a central processing unit (CPU).

The storage unit 132 stores data and a computer program. The storage unit 132 includes a main storage device and an auxiliary storage device. The main storage device is a semiconductor memory, for example. The auxiliary storage device is, for example, a semiconductor memory and/or a hard disk drive. The storage unit 132 may include a removable media.

The control unit 131 executes the computer program stored in the storage unit 132 to execute the operation of the separation device 100. The control unit 131 receives image data from the imaging unit 120.

For example, the control unit 131 determines, on the basis of the image data from the imaging unit 120, whether or not particles having a predetermined particle size or smaller have passed through the first recovery passage 21 or the second recovery passage 23. When particles having a predetermined particle size or smaller have passed through the first recovery passage 21 or the second recovery passage 23, there is a high possibility that the separation by the HDF 3 has not been performed normally. Therefore, upon determining that particles having the predetermined particle size or smaller have passed through the first recovery passage 21 or the second recovery passage 23, the control unit 131 stops driving the sample liquid sending portion 61, the transport liquid sending portion 63, and the recovered liquid sending portion 65.

Also, the control unit 131 determines, on the basis of the image data from the imaging unit 120, whether or not the particles have passed through the second recovery passage 23. When the particles do not pass through the second recovery passage 23, there is a high possibility that the separation by the HDF 3 or the separation by dielectrophoresis has not been normally performed. Therefore, upon determining that the particles have not passed through the second recovery passage 23, the control unit 131 stops driving the sample liquid sending portion 61, the transport liquid sending portion 63, and the recovered liquid sending portion 65. It is noted that the control unit 131 may determine, on the basis of the image data from the imaging unit 120, whether or not the particle has passed through the first recovery passage 21. Upon determining that the particles have not passed through the first recovery passage 21, the control unit 131 may stop driving the sample liquid sending portion 61, the transport liquid sending portion 63, and the recovered liquid sending portion 65.

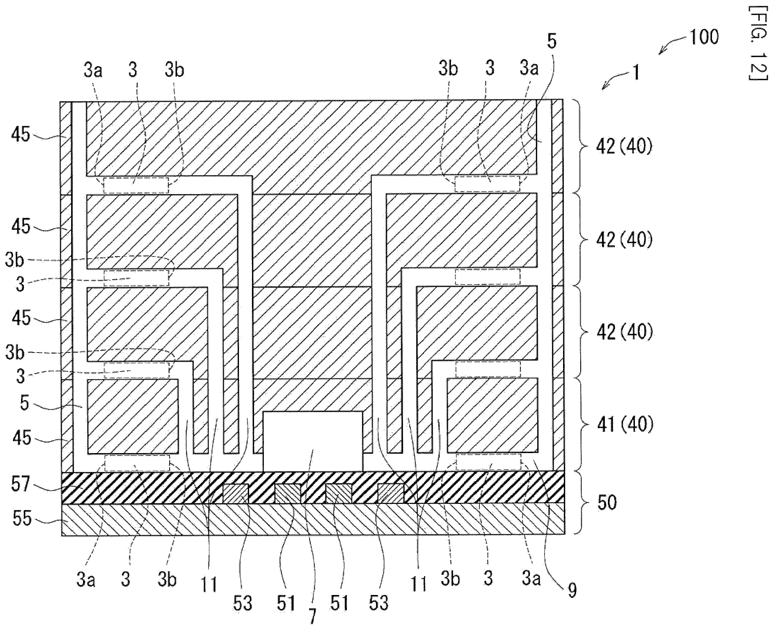

Next, the separation device 100 according to the first modified example of the present invention will be described with reference to FIG. 12. FIG. 12 is a cross-sectional view schematically illustrating a structure of a separation device 100 including a channel chip 1 according to the first modified example of the present invention. In the first modified example, unlike the preferred embodiment described above with reference to FIGS. 1 to 11, an example in which the plurality of filter layers 40 includes the two or more second filter layers 42 will be described.

As illustrated in FIG. 12, in the separation device 100 according to the first modified example of the present invention, the plurality of filter layers 40 include the first filter layer 41 and the two or more second filter layers 42 stacked on the first filter layer 41. In the first modified example, the plurality of filter layers 40 includes the first filter layer 41 and three second filter layers 42. Therefore, the channel chip 1 of the first modified example can process more sample liquid L1 than the channel chip 1 of the preferred embodiment described with reference to FIGS. 1 to 11. It is noted that the plurality of filter layers 40 may include the two second filter layers 42 or the four or more second filter layers 42.

Also, the channel chip 1 includes a plurality of the second connection channels 11 that connect the outlets 3b of the HDFs 3 of a plurality of the second filter layers 42 (here, three second filter layers 42) and the common channel 7. In the first modified example, the plurality of second connection channels 11 extend to the first filter layer 41 and are connected to the common channel 7 without joining each other in the second filter layers 42. Here, in a case where the second connection channels 11 are joined together in the second filter layers 42, high positioning accuracy is required when the second filter layers 42 are stacked. However, in the first modified example, since the second connection channels 11 are not joined together in the second filter layers 42, it is possible to suppress the need for high positioning accuracy when the second filter layers 42 are stacked. That is, it is possible to prevent the manufacturing of the channel chip 1 from becoming complicated. It is noted that the plurality of second connection channels 11 may be joined together in the second filter layers 42 and then connected to the common channel 7 in the first filter layer 41.

Other structures and effects of the first modified example are the same as those of the above preferred embodiment.

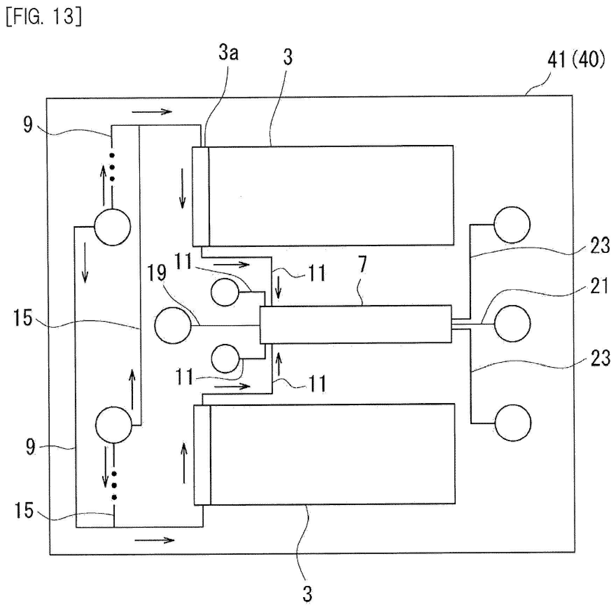

Next, the separation device 100 according to the second modified example of the present invention will be described with reference to FIG. 13. FIG. 13 is a plan view schematically illustrating a structure of the first filter layer 41 of the channel chip 1 according to the second modified example of the present invention. In the second modified example, unlike the preferred embodiment described above with reference to FIGS. 1 to 11, an example in which the first connection channel 9 and the third connection channel 15 are connected to the HDF 3 after being joined together will be described.

As illustrated in FIG. 13, in the channel chip 1 according to the second modified example of the present invention, the first connection channel 9 and the third connection channel 15 are joined together and then connected to the inlet 3a of the HDF 3. Therefore, since the path through which the first connection channel 9 and the third connection channel 15 pass can be simplified, the design of the channel chip 1 can be simplified.

Also, in the channel chip 1 according to the second modified example, the second connection channel 11 is connected such as to intersect the common channel 7. Specifically, the second connection channel 11 is connected substantially perpendicularly to the common channel **7.** For example, in a case where the second connection channel 11 is, for example, connected substantially perpendicular to the common channel 7 or connected such as to be inclined with respect to the common channel 7, it is possible to separate specific particles by dielectrophoresis as in the above-described preferred embodiment. It is noted that, although not illustrated, similarly to the first connection channel 9 and the third connection channel 15, the plurality of second connection channels 11 may be joined together and then connected to the common channel 7.

Other structures and effects of the second modified example are the same as those of the above preferred embodiment.

The preferred embodiments of the present disclosure have been described above with reference to the drawings. However, the present disclosure is not limited to the preferred embodiments described above, and can be implemented in various modes within the range not departing from the gist thereof. Also, by appropriately combining the plurality of constituent elements disclosed in the preferred embodiment described above, it is possible to form various inventions. For example, some constituent elements may be deleted from all constituent elements shown in the preferred embodiments. Further, the constituent elements of different preferred embodiments may be appropriately combined. The drawings schematically show the respective constituent elements mainly in order to facilitate understanding, and the thickness, the length, the number, the interval, etc., of each constituent element shown in the figures may be different from the actual ones for convenience in creating the drawings. Also, the material, the shape, the size, etc., of each constituent element shown in the preferred embodiments described above are not particularly limited but just an example, and can be variously changed within the range substantially not departing from the effects of the present disclosure.

For example, in the above preferred embodiments, an example has been described in which the plurality of filter layers 40 include the first filter layer 41 disposed on one surface of the dielectrophoretic layer 50 and the second filter layer 42 disposed on the first filter layer 41, but the present invention is not limited thereto. For example, the plurality of filter layers 40 may include the filter layer 40 disposed on one surface of the dielectrophoretic layer 50 and the filter layer 40 disposed on the other surface of the dielectrophoretic layer 50.

Also, in the above preferred embodiments, the example in which the common channel 7 is disposed in the first filter layer 41 disposed on one surface of the dielectrophoretic layer 50 among the plurality of filter layers 40 has been described, but the present invention is not limited thereto. For example, the common channel 7 may be disposed in any one of the one or more second filter layers 42 stacked on the first filter layer 41.

Also, in the above preferred embodiments, the example in which the common channel 7 is disposed in the filter layer 40 having the HDF 3 has been described, but the present invention is not limited thereto. For example, a layer which does not have the HDF 3 may be separately provided, and the common channel 7 may be disposed in the layer which does not have the HDF 3.

Also, in the above preferred embodiments, the example in which each filter layer 40 includes the plurality of HDFs 3 has been described, but the present invention is not limited thereto. For example, each filter layer 40 may have only one HDF 3.

Also, in the above preferred embodiments, the example in which the HDF 3 is disposed symmetrically with respect to the common channel 7 in each filter layer 40 has been described, but the present invention is not limited thereto. For example, the HDF 3 may be disposed symmetrically in each filter layer 40.

Also, in the above preferred embodiments, for example, in each filter layer 40, an example has been described in which the channel lengths of the plurality of first connection channels 9 are substantially the same and the channel lengths of the plurality of third connection channels 15 are substantially the same, but the present invention is not limited thereto. For example, in each filter layer 40, the channel lengths of the plurality of first connection channels 9 may be different. Also, in each filter layer 40, the channel lengths of the plurality of third connection channels 15 may be different.

Also, although not described in the above preferred embodiments, the recovered liquid sending portion 65 may be capable of adjusting the amount of the recovered liquid L3 to be supplied to the recovered liquid supply passage 17. That is, the recovered liquid sending portion 65 may be capable of adjusting the amount of the recovered liquid L3 flowing through the common channel 7. In this case, the amounts of the sample liquid L1 and the transport liquid L2 flowing through the common channel 7 are changed by changing the amount of the recovered liquid L3 flowing through the common channel 7. That is, the ease of flow of the sample liquid L1 and the transport liquid L2 with respect to the common channel 7 changes. Therefore, in the HDF 3 connected to the common channel 7, the ease of flow from the main channel 31 to the auxiliary channel 33 changes. Therefore, for example, even in a case where the dimensions such as the width and depth of the channel at each position of the HDF 3 deviate from the set values due to manufacturing variations, by adjusting the amount of the recovered liquid L3 made to flow to the recovered liquid supply passage 17, it is possible to suppress particles (for example, cancer cells and white blood cells) larger than a predetermined particle size from flowing to the auxiliary channel 33 or particles (for example, red blood cells and platelets) having a predetermined particle size or smaller from passing through the main channel 31 and flowing to the common channel 7. Therefore, even if the dimension of the channel deviates from the set value, particles having a predetermined particle size can be separated.

Also, in the above preferred embodiments, an example in which the control unit 131 stops driving the sample liquid sending portion 61, the transport liquid sending portion 63, and the recovered liquid sending portion 65 upon determining that particles having a predetermined particle size or smaller have passed through the first recovery passage 21 or the second recovery passage 23, that the particles have not passed through the second recovery passage 23, or that the particles have not passed through the first recovery passage 21 is described. However, the present invention is not limited thereto.

For example, upon determining that particles having a predetermined particle size or smaller have passed through the first recovery passage 21 or the second recovery passage 23, the control unit 131 may adjust the amount of the recovered liquid L3 flowing through the common channel 7 so that particles having the predetermined particle size or smaller do not pass through the first recovery passage 21 or the second recovery passage 23. Also, for example, upon determining that the particles have not passed through the second recovery passage 23, the control unit 131 may adjust the amount of the recovered liquid L3 flowing through the common channel 7 so that the particles pass through the second recovery passage 23. Also, for example, upon determining that the particles have not passed through the first recovery passage 21, the control unit 131 may adjust the amount of the recovered liquid L3 flowing through the common channel 7 so that the particles pass through the first recovery passage 21.

Also, for example, in the preferred embodiments described above, the example in which blood is used as the sample liquid L1 and the liquid culture media is used as the transport liquid L2 is described, but the present invention is not limited thereto. For example, a liquid other than blood may be used as the sample liquid L1 and a liquid other than the liquid culture media may be used as the transport liquid L2.

Also, in the preferred embodiments described above, the particles to be recovered from the outlet 21a (for example, the cancer cells) are exemplified as a target to be recovered, but the present invention is not limited to this. For example, particles or a liquid component to be recovered from the outlet 23a may be the target to be recovered.

Also, in the above preferred embodiments, an example in which the transport liquid L2 and the recovered liquid L3 do not contain particles is described, but the present invention is not limited thereto. The transport liquid L2 and the recovered liquid L3 may contain particles. However, it is preferable to separate the particles contained in the transport liquid L2 and the recovered liquid L3 from the particles contained in the sample liquid L1 by dielectrophoresis.

Also, in the above preferred embodiments, the example of using the transport liquid L2 has been described. That is, the example in which the transport liquid L2 is made to flow to the HDF 3 in addition to the sample liquid L1 is described. However, the present invention is not limited thereto. For example, only the sample liquid L1 may flow into the HDF 3. For example, seawater containing particles may be used as the sample liquid L1 to separate the particles from the seawater.

### Industrial Applicability

The present invention is applicable to the field of channel chips.

### Reference Signs List

1 : channel chip
3 : HDF (hydrodynamic filter)
3a : inlet
3b : outlet
5 : first common supply passage (common supply passage)
7 : common channel
9 : first connection channel (inlet-side connection channel)
11 : second connection channel (outlet-side connection channel)
21 : first recovery passage
23 : second recovery passage
40 : filter layer
41 : first filter layer
42 : second filter layer
50 : dielectrophoretic layer
51, 53 : electrode
L1 : sample liquid

## Claims

1. A channel chip comprising:
a plurality of filter layers each having a hydrodynamic filter;
a common supply passage that supplies a sample liquid to the hydrodynamic filters of the plurality of filter layers;
a common channel through which a liquid that has passed through the plurality of hydrodynamic filters passes; and
a dielectrophoretic layer having electrodes that cause dielectrophoresis of dielectric particles contained in the liquid passing through the common channel,
wherein the plurality of filter layers and the dielectrophoretic layer are stacked.

2. The channel chip according to claim 1, wherein the plurality of filter layers include a first filter layer stacked on the dielectrophoretic layer and one or more second filter layer stacked on the first filter layer, and
the common channel is disposed in the first filter layer.

3. The channel chip according to claim 2, wherein the plurality of filter layers include a plurality of the second filter layers,
the channel chip includes a plurality of outlet-side connection channels connecting outlets of the hydrodynamic filters of the plurality of second filter layers and the common channel, and
the plurality of outlet-side connection channels extend to the first filter layer and are connected to the common channel without joining together in the second filter layers.

4. The channel chip according to any one of claims 1 to 3, wherein each of the filter layers has the plurality of hydrodynamic filters.

5. The channel chip according to claim 4, wherein the plurality of hydrodynamic filters of each of the filter layers are disposed symmetrically with respect to the common channel.

6. The channel chip according to claim 4 or 5, wherein the channel chip includes a plurality of inlet-side connection channels that connect the common supply passage and inlets of the plurality of hydrodynamic filters in each of the filter layers, and
in each of the filter layers, the plurality of inlet-side connection channels has substantially the same channel length.

7. The channel chip according to any one of claims 4 to 6, further comprising a first recovery passage and a pair of second recovery passages connected to a downstream portion of the common channel,
wherein the pair of second recovery passages are disposed such as to sandwich the first recovery passage, and
the dielectric particles separated from the liquid that has passed through the hydrodynamic filter flow through the first recovery passage.
